# EUROPÄISCHE PATENTSCHRIFT

(11) **EP 0 702 953 B1**
(45) Veröffentlichungstag und Bekanntmachung des Hinweises auf die Patenterteilung: **02.02.2000**
(21) Anmeldenummer: 95112892.5
(22) Anmeldetag: 17.08.1995
(51) Int. Cl.: A61K 31/385

(54) **Darreichungsformen enthaltend feste Salze von R-Thioctsäure mit verbesserter Freisetzung und Bioverfügbarkeit**
Dosage form comprising solid salts of R-alpha-lipoic acid with improved bioavailability
Forme de dosage à biodisponibilité améliorée comprenant des sels solides de la R-acide alpha-lipoique ou des sels solides

(30) Priorität: 22.09.1994 DE 4433764
(43) Veröffentlichungstag der Anmeldung: 27.03.1996
(60) Teilanmeldung: 99110836.6
(73) Patentinhaber: ASTA Medica Aktiengesellschaft, 01277 Dresden (DE)
(72) Erfinder: Hettche, Helmut, Dr., D-63128 Dietzenbach (DE); Rischer, Matthias, Dr., D-63477 Maintal (DE); Sarlikiotis, Werner, Dr., D-60316 Frankfurt (DE)
(74) Vertreter: Decker, Karl-Ludwig

(56) Entgegenhaltungen:
- EP-A- 0 427 246
- EP-A- 0 427 247
- EP-A- 0 530 446
- DE-A- 4 317 646
- DE-A- 4 343 593

## Beschreibung

Thioctsäure (= α-Liponsäure) ist chemisch 1,2-Dithiacyclopentan-3-valeriansäure. Die Herstellung der freien R-Thioctsäure ist beispielsweise in der DE-OS 41 37 773 beschrieben.

Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen α-Ketosäuren. Thioctsäure wird seit längerer Zeit bei verschiedenen Erkrankungen eingesetzt, so unter anderem bei Lebererkrankungen, bei Leberschädigungen durch Pilzvergiftung sowie bei diabetischer und alkoholischer Polyneuropathie, einer mit Stoffwechselerkrankungen einhergehenden Veränderung peripherer Nerven.

Die vorliegende Erfindung betrifft Arzneimittelformulierungen enthaltend Thioctsäure oder feste Salze von Thioctsäure mit verbesserter Bioverfügbarkeit.

Die Erfindung bezieht sich nicht nur auf die racemische Form, sondern ebenso auf die reine (R)-beziehungsweise (S)-Thioctsäure sowie auf Gemische von (R)- und (S)-Thioctsäure mit beliebiger Zusammensetzung.

Von dem reinen optischen Isomeren der Thioctsäure (R- und S-Form, das heißt R-Thioctsäure und S-Thioctsäure) sind im Gegensatz zu dem Racemat das R-Enantiomer vorwiegend antiphlogistisch und das S-Enantiomer vorwiegend antinociceptiv wirksam, wobei die antiphlogistische Wirkung des R-Enantiomeren beispielsweise um einen Faktor 10 stärker ist als diejenige des Racemats.

Die antinociceptive (analgetische) Wirkung des S-Enantiomeren ist beispielsweise um einen Faktor bis 6 stärker als diejenige des Racemats.

Die Enantiomeren stellen daher im Vergleich zu dem Racemat sehr viel spezifischere und stärker wirksame Wirkstoffe dar.

Die Wirkungen sind in EP 427 246 und EP 427 247 sowie im GbM 90 17 987.0 und EP 530 446 beschrieben.

Eine Kombination von Thioctsäure mit Vitaminen ist in EP 572 922 beschrieben.

R,S-Thioctsäure hat einen Schmelzpunkt von 60,5°C. R-Thioctsäure hat einen Schmelzpunkt von 50,6 - 50,7°C. Beide sind bei 25°C in Wasser zu 12,14 mg/10 ml, in Methanol, Ethanol, Chloroform, Dimethylformamid und n-Octanol zu mehr als 1000 mg/10 ml löslich.

Oral anzuwendende Arzneiformen haben im Vergleich zu Parenteralia üblicherweise einen Preisvorteil, der sich günstig auf die Tagestherapiekosten auswirkt. Die bisherigen Darreichungsformen der Thioctsäure haben jedoch den Nachteil, eine relativ niedrige Bioverfügbarkeit aufzuweisen. Eine niedrige Bioverfügbarkeit bedeutet, daß bei peroraler Gabe der Darreichungsform im Blut eines Probanden oder Patienten im Vergleich zur intravenösen Gabe relativ wenig des unveränderten Wirkstoffs aufgefunden wird. Dies hat zur Folge, daß bei peroraler Gabe die Wirksamkeit der Medikation nicht das Ausmaß erreichen kann, wie es bei der intravenösen Gabe der Wirksubstanz der Fall ist. Es besteht daher die Aufgabe, Darreichungsformen zu entwickeln, die bei guter Lagerstabilität eine möglichst hohe Bioverfügbarkeit aufweisen.

Gegenstand der vorliegenden Erfindung sind daher Darreichungsformen enthaltend R-Thioctsäure, deren Bioverfügbarkeit gegenüber denen der bisherigen Darreichungsformen erhöht ist.

Der Wirkstoff kann auch in eine magensaftresistente Form gebracht werden, die sich nach dem Übergang in das Duodenum bei dem pH-Wert des Darmsaftes schnell auflöst. Der pH-Wert des Darmsafts beträgt 6,8 - 7,3.

Als Wirkstoff kann hierbei entweder die freie Thioctsäure oder - noch vorteilhafter - ein Salz der Thioctsäure eingesetzt werden.

Nachteilig ist die schlechte Freisetzung des Wirkstoffes R-Thioctsäure aus den daraus hergestellten Darreichungsformen. Bei der Freisetzungsprüfung nach dem Deutschen Arzneibuch, 10. Ausgabe (Blattrührermethode) oder der USP XXII (mit Apparatus 2) ist die Freisetzung des Wirkstoffes aus Tabletten entsprechend Beispiel 2a bei Verwendung von 0,06 N HCl als Freisetzungsmedium bei 37°C wie folgt:

| | |
|---|---|
| Nach 15 Minuten | 6% |
| Nach 30 Minuten | 9% |
| Zerfallzeit | < 2 min |

Dies steht im Gegensatz zum Verhalten von Tabletten aus dem Racemat der Thioctsäure, die bei gleicher Zusammensetzung der Tabletten folgende Freisetzung des Wirkstoffes zeigen (Methode wie oben):

| | |
|---|---|
| Nach 15 Minuten | 99% |
| Nach 30 Minuten | 100% |
| Zerfallzeit | 2,5 min |

Überraschend zeigte es sich, daß bei Verwendung der festen Salze der R-Thioctsäure zur Herstellung der Tabletten entsprechend Beispiel 3 wieder gute Freisetzungswerte erhalten werden (Methode wie oben):

| | |
|---|---|
| Nach 15 Minuten | 75% |
| Nach 30 Minuten | 88% |
| Zerfallzeit | < 1 min |

Als Salzbildner kommen zum Beispiel übliche Basen beziehungsweise Kationen in Frage, die in der Salzform physiologisch verträglich sind. Beispiele hierfür sind: Alkali- oder Erdalkalimetalle, wie beispielsweise Natrium, Kalium, Calcium. Magnesium, Ammoniumhydroxid, basische Aminosäuren wie beispielsweise Ornithin, Cystin, Methionin, Arginin und Lysin, Amine der Formel N R1 R2 R3 ,worin die Reste R1, R2 und R3 gleich oder verschieden sind und Wasserstoff, C1-C4-Alkyl, wie beispielsweise Methyl, Ethyl, Propyl, Isopropyl, Butyl, Isobutyl ,tert.-Butyl, oder C1-C4-Oxyalkyl bedeuten, wie beispielsweise Mono- und Diethanolamin, 1-Amino-2-propanol, 3-Amino-1-propanol; Alkylendiamine mit einer Alkylenkette aus 2 bis 6-C-Atomen wie beispielsweise Ethylendiamin oder Hexamethylentetramin, gesättigte cyclische Aminoverbindungen mit 4-6 Ringkohlenstoffatomen wie beispielsweise Piperidin, Piperazin, Pyrrolidon, Morpholin; N-Methylglucamin, Kreatin und Trometamol

Die Salzbildner sind in den oben genannten Patentschriften bereits allgemein aufgeführt, ohne daß jedoch auf die Herstellung der Salze und deren besondere Eignung für die Herstellung von bestimmten Darreichungsformen eingegangen wird.

Flüssige Darreichungsformen mit dem Trometamol-, Lysin- und Ethylendiaminsalz der R-Thioctsäure sind in verschiedenen Patentanmeldungen beschrieben, beispielsweise in EP 427 246 EP 427 247, EP 530 446.

Hierbei handelt es sich jedoch ausnahmslos um Lösungen, bei denen das Salz durch Zusammengeben von R-Thioctsäure und der entsprechenden Base in Lösung gebildet wird. Das Salz selbst wird dabei nicht isoliert, tritt also in kristalliner Form nicht auf. Darüber hinaus wird regelmäßig die Base im Überschuß eingesetzt, so daß ein reines Salz nicht isolierbar wäre.

Salze von R-Thioctsäure mit L-Lysin und L-Arginin sind im spanischen Patent 313 056 erwähnt. Es fehlen jedoch Angaben zur Herstellung und Charakterisierung der Salze und der Hinweis auf die bessere Freisetzung und Bioverfügbarkeit des Wirkstoffes aus daraus hergestellten Darreichungsformen.

Gegenstand der Erfindung sind weiterhin alle Darreichungsformen, enthaltend feste Salze der R-Thioctsäure, die sich durch eine bessere Wirkstofffreisetzung und bessere Bioverfügbarkeit auszeichnen als Darreichungsformen aus R-Thioctsäure.

Die Darreichungsformen aus Salzen der R-Thioctsäure haben gegenüber den Darreichungsformen aus freier R-Thioctsäure nicht nur den Vorteil der besseren Freisetzung und Bioverfügbarkeit des Wirkstoffes, sondern darüber hinaus auch den Vorteil der leichteren Herstellbarkeit:
Bei der Herstellung von Darreichungsformen aus R-Thioctsäure kommt es durch den niedrigeren Schmelzpunkt des Wirkstoffs von 50,6 - 50,7°C sowohl bei der Herstellung von Formlingen als auch bei deren Weiterverarbeitung, beispielsweise beim Auftragen eines geschmacksschützenden Überzugs, leicht zum Sintern des Wirkstoffs. Das Sintern des Wirkstoffs, beispielsweise bei der Anwendung von leicht erhöhten Temperaturen, wie sie zum Trocknen und Verfestigen des Überzugs erforderlich sind, führt zur Reduzierung der Porosität , im Extremfall sogar zum völligen Durchsintern des Formlings. Dies führt dazu, daß sich der Formling (Tablette, Pellet, Granulat) nur noch extrem langsam im Magen-Darmtrakt auflöst. Daraus resultiert ebenfalls eine schlechte Bioverfügbarkeit des Wirkstoffs. Bei der Verwendung der Salze der R-Thioctsäure kommt es nicht zum Sintern und damit auch nicht zu einer Verschlechterung der Bioverfügbarkeit.

Darüber hinaus führt die Neigung zum Sintern der freien R-Thioctsäure bei der Tablettenpressung zum Kleben der Tabletten am Stempelwerkzeug, wenn Tabletten mit einem höheren Gehalt an Wirkstoff im Produktionsmaßstab hergestellt werden sollen. Dies kann soweit gehen, daß der Preßvorgang abgebrochen werden muß.

Die gleichzeitige Lösung der Probleme der schlechten Wirkstofffreisetzung und der komplizierten Herstellung der Darreichungsformen durch Verwendung der Salze der R-Thioctsäure anstatt der freien R-Thioctsäure ist überraschend.

Es muß dabei darauf hingewiesen werden, daß die beiden Probleme des Tablettenzerfalls und der Freisetzung nicht direkt miteinander verknüpft sind: Einwandfreie Tabletten ohne Sinterungserscheinungen zeigen trotz guter Zerfallseigenschaften eine schlechte Freisetzung des Wirkstoffes R-Thioctsäure, wie anhand des obigen Beispiels dargestellt wurde.

Als Darreichungsformen für die Salze der R-Thioctsäure können nicht nur Tabletten, Granulate, Inhalationspulver, Hartgelatinekapseln und Pellets aufgeführt werden, sondern auch Weichgelatinekapseln, Dosieraerosole, Suspensionen, Salben und Suppositorien, bei denen das Salz in einer Grundlage inkorporiert ist, beispielsweise in amphiphilen oder lipophilen Medien, Polyethylenglykol oder Treibmitteln.

Bei den letztgenannten Darreichungsformen kann im Vergleich zur R-Thioctsäure ebenfalls eine bessere Freisetzung aus der Grundlage beobachtet werden.

Gegenstand der Erfindung sind weiterhin Darreichungsformen, enthaltend feste Salze der R-Thioctsäure. Die Darreichungsformen können entweder als Arzneimittel oder als Lebensmittelzusatzstoff Verwendung finden. Die Salze können aus den oben beschriebenen Salzbildnern und R-Thioctsäure gebildet werden. Besonders bevorzugt sind die Salze aus R-Thioctsäure und Trometamol, L-Lysin, L-Arginin, Natriumhydroxid, Ammoniumhydroxid und Creatin. Die Herstellung erfolgt nach allgemein in der Technik bekannten Methoden, wie sie im Zusammenhang mit dem Racemat der Thioctsäure beispielsweise in DE-OS 16 95 358, dem französischen Patent 4630 M oder Beispiel 1 beschrieben sind.

Die analytischen Kennzahlen einiger der als besonders bevorzugt geltend 116 - 118 °C en Salze sind:

| **Säure** | **Salzbildner** | **Schmelzpunkt** |
|---|---|---|
| R-Thioctsäure | Trometamol | 116 - 118 °C |
| | Natriumhydroxid | 247 - 257 °C (Zersetzung) |
| | ohne (freie Säure) | 50,6 - 50,7 °C |

Bei Einsatz von optisch aktiven Salzbildnern kann ein weiterer Vorteil genutzt werden:
Es ist möglich, racemische Thioctsäure mit dem optisch aktiven Salzbildner umzusetzen zu den R- und S-Thioctsäure-Salzen. Diese können aufgrund ihrer unterschiedlichen Eigenschaften getrennt und anschließend direkt zu den Darreichungsformen weiterverarbeitet werden, wobei sich eine Freisetzung der R- beziehungsweise S-Thioctsäure erübrigt. Als Salzbildner können beispielsweise eingesetzt werden: L-Lysin, L-Arginin, D(-)-N-Methylglucamin.

Die Herstellung der Darreichungsformen erfolgt nach den hierfür üblichen Standardmethoden, wie sie beispielsweise in dem Standardwerk Sucker, Fuchs, Speiser Pharmazeutische Technologie, Thieme Verlag Stuttgart, 1978, beschrieben sind.

Die Magensaftresistenz der Darreichungsformen wird entweder erzielt durch Einbettung des Wirkstoffs in anionische Polymere beispielsweise mit einem pKₛ-Wert von 5,0 bis 5,5 und nachfolgende Herstellung entsprechender Darreichungsformen unter Verwendung dieser Einbettung. Die übliche Methode zur Erzielung einer Magensaftresistenz von Formulierungen ist jedoch deren Überziehung mit anionischen Polymeren beispielsweise mit einem pKₛ-Wert von 5,0 bis 5,5, die sich erst bei einem pH-Wert von minimal 5,0 beginnen aufzulösen.

Als solche Substanzen wären beispielsweise zu nennen: Schellack Hydroxypropylmethylcellulosephthalat (Handelsprodukt beispielsweise HP 55/Hersteller: Shinetsu Chemical Company, Tokio), Celluloseacetatphthalat (Handelsprodukt beispielsweise Aquateric/Hersteller: FMC Export Corporation, Philadelphia, USA), Polyvinylacetatphthalat, Copolymerisate aus Methacrylsäure und Methacrylsäureestern (Handelsprodukte beispielsweise Eudragit L und Eudragit S-Typen sowie Eudragit L 30 D/Hersteller: Rhöm Pharma). Die Herstellung der Darreichungsformen erfolgt nach den hierfür üblichen Standardmethoden, wie sie beispielsweise in dem Standardwerk Sucker, Fuchs, Speiser Pharmazeutische Technologie, Thieme Verlag Stuttgart 1978, beschrieben sind sowie in Bauer, Lehmann, Osterwald Rothgang Überzogene Arzneiformen, Wissenschaftliche Verlagsgesellschaft mbH Stuttgart, 1988.

Die Prüfung auf Magensaftresistenz erfolgt nach der Methode der USP XXII, <701> Disintegration, wobei die Darreichungsformen eine Resistenz gegenüber künstlichem Magensaft über zwei Stunden zeigen müssen, mindestens jedoch über 30 Minuten.

### Beispiel 1:

### Synthese von Salzen der Thioctsäure

1 Äquivalent der Base wird in Ethanol suspendiert, auf ca. 50 °C erwärmt und unter Rühren mit 1 Äquivalent Thioctsäure versetzt. Anschließend wird noch kurz nachgerührt, die Lösung abgekühlt, wobei das Salz langsam auskristallisiert. Nach vollständiger Kristallisation wird das Salz abfiltriert und im Vakuum getrocknet.

### Beispiel 2:

### Tabletten mit 221,3 mg Natriumsalz der R-Thioctsäure, entsprechend 200 mg R-Thioctsäure

224,00 g Natriumsalz der R-Thioctsäure, 168,00 g Lactose, 83,40 g mikrokristalline Cellulose, 30,24 g Maisstärke und 10,08 g Poly(1vinyl-2-pyrrolidon) werden gesiebt, gemischt und mit gereinigtem Wasser befeuchtet. Anschließend wird das befeuchtete Gemisch über ein Lochsieb granuliert. Im Anschluß daran wird das feuchte Granulat im Wirbelschichtgranulator getrocknet. Danach werden 35,32 g mikrokristalline Cellulose, 2,24 g hochdisperses Siliciumdioxid und 6,72 g Magnesiumstearat gesiebt und mit dem trockenen Granulat vermischt. Die so entstandene Preßmasse wird mit Hilfe einer Tablettiermaschine zu Tabletten verpreßt.

Eine Tablette wiegt 553,3 mg und enthält 221,3 mg Natriumsalz der R-Thioctsäure, entsprechend 200 mg R-Thioctsäure.

### Beispiel 2a:

### Vergleichsbeispiel: Tabletten mit 200 mg R-Thioctsäure

224,00 g R-Thioctsäure, 168,00 g Lactose, 83,40 g mikrokristalline Cellulose, 30,24 g Maisstärke und 10,08 g Poly(1vinyl-2-pyrrolidon) werden gesiebt, gemischt und mit gereinigtem Wasser befeuchtet. Anschließend wird das befeuchtete Gemisch über ein Lochsieb granuliert. Im Anschluß daran wird das feuchte Granulat im Wirbelschichtgranulator getrocknet. Danach werden 35,32 g mikrokristalline Cellulose, 2,24 g hochdisperses Siliciumdioxid und 6,72 g Magnesiumstearat gesiebt und mit dem trockenen Granulat vermischt. Die so entstandene Preßmasse wird mit Hilfe einer Tablettiermaschine zu Tabletten verpreßt.

Eine Tablette wiegt 500,0 mg und enthält 200,0 mg R-Thioctsäure.

### Beispiel 3:

### Tabletten mit 317,4 mg Trometamolsalz der R-Thioctsäure, entsprechend 200 mg R-Thioctsäure.

224,00 g Trometamolsalz der R-Thioctsäure, 168,00 g Lactose, 83,40 g mikrokristalline Cellulose, 30,24 g Maisstärke und 10,08 g Poly(1vinyl-2-pyrrolidon) werden gesiebt, gemischt und mit gereinigtem Wasser befeuchtet. Anschließend wird das befeuchtete Gemisch über ein Lochsieb granuliert. Im Anschluß daran wird das feuchte Granulat im Wirbelschichtgranulator getrocknet. Danach werden 35,32 g mikrokristalline Cellulose, 2,24 g hochdisperses Siliciumdioxid und 6,72 g Magnesiumstearat gesiebt und mit dem trockenen Granulat vermischt. Die so entstandene Preßmasse wird mit Hilfe einer Tablettiermaschine zu Tabletten verpreßt.

Eine Tablette wiegt 793,5 mg und enthält 317,4 mg Trometamolsalz der R-Thioctsäure, entsprechend 200 mg R-Thioctsäure.

### Beispiel 4:

### Hartgelatinekapseln mit 317,4 mg Trometamolsalz der R-Thioctsäure, entsprechend 200 mg R-Thioctsäure

Zunächst werden 3,34 g Hydroxypropylcellulose in 34 g gereinigtes Wasser gegeben und unter Rühren bei Raumtemperatur gelöst. Die Lösung wird über Nacht bei Raumtemperatur stehengelassen. Diese Lösung ist die Granulierlösung. Danach werden 100,00 g Trometamolsalz der R-Thioctsäure und 6,66 g Hydroxypropylcellulose, niedrig substituiert, gesiebt, gemischt und mit der Granulierlösung befeuchtet. Anschließend wird das befeuchtete Gemisch über ein Lochsieb granuliert. Im Anschluß daran wird das feuchte Granulat im Wirbelschichtgranulator getrocknet. Danach werden 4,00 g Magnesiumstearat gesiebt und mit dem trockenen Granulat vermischt. Die so entstandene Kapselmasse wird mit Hilfe einer Kapselmaschine zu 361,8 mg in Hartgelatinekapseln Größe 1 gefüllt. Eine Kapsel enthält 317,4 mg Trometamolsalz der R-Thioctsäure, entsprechend 200 mg R-Thioctsäure

### Beispiel 5:

### Inhalationspulver mit 15,87 mg Trometamolsalz der R-Thioctsäure, entsprechend 10 mg R-Thioctsäure

158,7 g Trometamolsalz der R-Thioctsäure, mikronisiert, werden mit 200 g Lactose- Monohydrat der mittleren Korngröße von 100 µm gemischt und die Mischung zu 35,87 mg in Hartgelatinekapseln der Größe 2 gefüllt.

Die Kapseln werden in handelsübliche Pulverinhalatoren eingelegt und durch die Einatemluft des Patienten entleert.

Eine Kapsel enthält 15,87 mg Trometamolsalz der R-Thioctsäure,
entsprechend 10 mg R-Thioctsäure.

### Beispiel 6:

### Weichgelatinekapseln mit 158,7 mg Trometamolsalz der R-Thioctsäure, entsprechend 100 mg R-Thioctsäure

Aus 2100 g mittelkettigen Triglyceriden (Handelsname: Miglyol 812-Neutralöl; Hersteller: Dynamit Nobel) und 700 g Hartfett (Handelsname: Witepsol H 15; Hersteller: Dynamit Nobel) wird eine Lösung hergestellt. In diese Lösung werden 2000 g Trometamolsalz der R-Thioctsäure gleichmäßig eingearbeitet. Die erhaltene Suspension wird in üblicher Weise in Weichgelatinekapseln gefüllt, indem pro Kapsel 380,9 mg der Suspension eingesetzt werden.

Eine Kapsel enthält 158,7 mg Trometamolsalz der R-Thioctsäure.

### Beispiel 7:

### Orale Suspension mit 7,94 % Trometamolsalz der R-Thioctsäure, entsprechend 5 % R-Thioctsäure

In 800 g mittelkettige Triglyceride (Handelsname Miglyol 812-Neutralöl Hersteller: Dynamit Nobel) werden 65 g Hochdisperses, amorphes, hydrophobes Siliciumdioxid (Handelsname Aerosil 972; Hersteller Degussa, Frankfurt), 1 g gemahlenes Saccharin-Natrium und 3 g gemahlenes Natriumcyclamat eingerührt und homogenisiert.

Nach Zusatz von 1 g Schokoladen- und 1 g Anisaroma werden 79,4 g Trometamolsalz der R-Thioctsäure eingerührt und homogenisiert.

Anschließend wird mit mittelkettigen Triglyceriden auf 1033 g, entsprechend 1 Liter aufgefüllt und gemischt.

5 ml der Suspension enthalten 397 mg Trometamolsalz der R-Thioctsäure, entsprechend 250 mg R-Thioctsäure.

### Beispiel 8:

### Suppositorien mit 221,3 mg Natriumsalz der R-Thioctsäure, entsprechend 200 mg R-Thioctsäure

404,2 g Natriumsalz der R-Thioctsäure werden in 3,376 kg geschmolzenem Hartfett* (s. Europäisches Arzneibuch) suspendiert. Nach Homogenisierung wird die Suspension in üblicher Weise in Hohlzellen von 2,3 ml ausgegossen und abgekühlt.
*) Hartfett ist ein Gemisch von Mono-, Di-, und Triglyceriden der gesättigten Fett- säuren von C₁₀H₂₀O₂ bis C₁₈H₃₆O₂.

Ein Suppositorium vom Gewicht 2,07 g enthält 221,3 mg Natriumsalz der R-Thioctsäure, entsprechend 200 mg R-Thioctsäure

### Beispiel 9:

### Salbe mit 1,11 % Natriumsalz der R-Thioctsäure,entsprechend 1 % R-Thioctsäure

779,5 g weißes Vaselin und 30 g Polyoxyethylen-20-stearylether (Handelsname: Brij® 78) werden bei einer Temperatur von etwa 75°C zusammengeschmolzen. 10,89 g Natriumsalz der R-Thioctsäure werden in 161 g dickflüssigem Paraffin suspendiert. Dieser Suspension wird die oben erhaltene Schmelze unter Rühren zugegeben. Anschließend wird die Salbe unter Rühren auf Raumtemperatur abgekühlt.

### Beispiel 10:

### Dosieraerosol mit 3,97 mg Trometamolsalz der R-Thioctsäure,entsprechend 2,5 mg R-Thioctsäure pro Hub

1000 g 2 H-Heptafluorpropan (= Treibmittel 227) werden auf eine Temperatur von etwa -55°C abgekühlt und unter Rühren mit einer Lösung aus 11,7 g Polyoxyethylen- 25-glyceryltrioleat (Handelsname: Tagat TO, Goldschmidt AG) in 11,7 g absolutem Ethanol versetzt. Anschließend werden 33,2 g mikronisiertes Trometamolsalz der R- Thioctsäure, 0,9 g mikronisiertes Saccharin Natrium und 6,75 g Pfefferminzöl zugesetzt und die entstandene Suspension homogenisiert. Unter weiterem Rühren und Kühlen wird die Suspension mit gekühltem Treibmittel 227 auf 1170,0 g aufgefüllt und sodann in Metalldosen abgefüllt, die mit Dosierventilen verschlossen werden, welche pro Hub 100 µl der Suspension freisetzen. Pro Hub werden damit 3,97 mg Trometamolsalz der R-Thioctsäure, entsprechend 2,5 mg R-Thioctsäure freigesetzt.

### Beispiel 11:

### Pellets mit 317,4 mg Trometamolsalz der R-Thioctsäure, entsprechend 200 mg R-Thioctsäure

Zunächst werden 634,8 g Trometamolsalz der R-Thioctsäure, 33,2 g Natriumcarboxymethylcellulose und 530,0 g mikrokristalline Cellulose gesiebt, gemischt und mit 500,0 g Wasser befeuchtet und angeteigt. Anschließend wird das befeuchtete Gemisch im Extruder zu Strängen geformt, die im Anschluß daran im Spheronizer zu kurzen Zylindern gebrochen und zu Pellets ausgerundet werden. Danach werden die feuchten Pellets im Wirbelschichtgranulator getrocknet.

Die so entstandenen Pellets können mit Hilfe einer Kapselmaschine zu 599 mg in Hartgelatinekapseln Größe 0 gefüllt werden. Eine Kapsel enthält 317,4 mg Trometamolsalz der R-Thioctsäure, entsprechend 200 mg R-Thioctsäure

## Patentansprüche

1. Darreichungsformen, enthaltend feste Salze von R-Thioctsäure.

2. Darreichungsform nach Anspruch 1, dadurch gekennzeichnet, daß sie feste Salze von R-Thioctsäure zusammen mit üblichen Trägern, Hilfsstoffen und/oder Verdünnungsmitteln enthält.

3. Darreichungsform nach Anspruch 1 oder 2, dadurch gekennzeichnet, daß sie feste Salze von R-Thioctsäure mit einer Base, ausgewählt aus Alkali- oder Erdalkalimetallen, Ammoniumhydroxid, basischen Aminosäuren wie Ornithin, Cystin, Methionin, Arginin und Lysin, Aminen der Formel N R1 R2 R3, worin die Reste R1, R2 und R3 gleich oder verschieden sind und Wasserstoff, C1-C4-Alkyl oder C1-C4-Oxyalkyl bedeuten, Alkylendiaminen mit einer Alkylenkette aus 2 bis 6-C-Atomen wie Ethylendiamin oder Hexamethylentetramin, Pyrrolidon, Morpholin; N-Methylglucamin, Kreatin und Trometamol, enthält.

4. Darreichungsform nach einem der Ansprüche 1 bis 3, dadurch gekennzeichnet, daß sie das feste Tromethamolsalz der R-Thioctsäure enthält.

5. Darreichungsform nach einem der Ansprüche 1 bis 4 zur Verwendung als Arzneimittel oder Lebensmittelzusatzstoff.

## Claims

1. Presentations containing solid salts of R-thioctic acid.

2. Presentation according to Claim 1, characterized in that it contains solid salts of R-thioctic acid together with conventional vehicles, ancillary substances and/or diluents.

3. Presentation according to Claim 1 or 2, characterized in that it contains solid salts of R-thioctic acid with a base selected from alkali metals or alkaline earth metals, ammonium hydroxide, basic amino acids such as ornithine, cystine, methionine, arginine and lysine, amines of the formula N R1 R2 R3, in which the radicals R1, R2 and R3 are identical or different and denote hydrogen, C1-C4-alkyl or C1-C4-oxyalkyl, alkylenediamines with an alkylene chain of 2 to 6 C atoms such as ethylenediamine or hexamethylenetetramine, pyrrolidone, morpholine; N-methylglucamine, creatine and trometamol.

4. Presentation according to any of Claims 1 to 3, characterized in that it contains the solid tromethamol salt of R-thioctic acid.

5. Presentation according to any of Claims 1 to 4 for use as pharmaceutical or food additive.

## Revendications

1. Formes d'administration contenant des sels solides d'acide R-thiooctique.

2. Forme d'administration selon la revendication 1,
caractérisée en ce qu'
elle renferme des sels solides d'acide R-thiooctique conjointement à des supports, des adjuvants et/ou des agents de dilution usuels.

3. Forme d'administration selon la revendication 1 ou 2,
caractérisée en ce qu'
elle renferme des sels solides d'acide R-thiooctyique avec une base choisie parmi les métaux alcalins ou alcalino-terreux, l'hydroxyde d'ammonium, les aminoacides basiques comme l'ornithine, la cystine, la méthionine, l'arginine et la lysine, des amines de formule,
N R1 R2 R3
dans laquelle les radicaux R1, R2 et R3 sont identiques ou différents et signifient de l'hydrogène, un alkyle en C1, C4, ou un oxyalkyle en C1-C4, des alkylène-diamines ayant une chaîne alkylène à base de 2 à 6 atomes de carbone comme l'éthylène-diamine ou l'hexaméthylènetétramine, la pyrrolidone, la morpholine, la N-méthylglucamine, la créatine, et le trométamol.

4. Forme d'administration selon l'une des revendications 1 à 3,
caractérisée en ce qu'
elle renferme le sel solide de trométamol d'acide R-thiooctique.

5. Forme d'administration selon l'une des revendications 1 à 4, en vue de l'utilisation en tant que médicament ou comme additif de produit alimentaire.
